# EUROPÄISCHE PATENTSCHRIFT

(11) **EP 0 877 957 B1**
(45) Veröffentlichungstag und Bekanntmachung des Hinweises auf die Patenterteilung: **26.06.2002**
(21) Anmeldenummer: 97901011.3
(22) Anmeldetag: 13.01.1997
(51) Int. Cl.: G01T 1/164, G01T 1/00

(54) **GAMMA-KAMERA**
GAMMA CAMERA
CAMERA GAMMA

(30) Priorität: 30.01.1996 DE 19603212
(43) Veröffentlichungstag der Anmeldung: 18.11.1998
(73) Patentinhaber: Forschungszentrum Karlsruhe GmbH, 76133 Karlsruhe (DE)
(72) Erfinder: DOERFEL, Hans, D-76227 Kandel (DE)
(74) Vertreter: Rückert, Friedrich, Dr.
(86) Internationale Anmeldenummer: EP9700124
(87) Internationale Veröffentlichungsnummer: WO9728463

(56) Entgegenhaltungen:
- US-A- 3 855 473
- US-A- 4 010 373
- US-A- 4 348 591
- US-A- 5 317 158
- IEEE NUCLEAR SCIENCE, Bd. 43, Nr. 3, Oktober 1995, USA, Seiten 1827-1831, XP002030397 REDUS,R ET AL.: "an imaging nuclear survey system"

## Beschreibung

Die Erfindung betrifft eine Gamma-Kamera nach dem Oberbegriff des Patentanspruchs 1, wie sie aus Hermann, H.J., Nuklearmedizin, Verlag Urban & Schwarzenberg München, 3. Auflage 1992 bekannt ist. Herkömmliche Gamma-Kameras bestehen im allgemeinen aus einem dünnen großflächigen NaI(Tl)-Kristall, an dessen Vorderseite ein Kollimator und an dessen Rückseite eine Anordnung von bis zu 90 Photomultipliern angebracht sind. Die Empfindlichkeit und das räumliche Auflösungsvermögen wird in erster Linie durch die Fläche des NaI(Tl)-Kristalls und die Struktur des Kollimators bestimmt. Das beste Ortsauflösungsvermögen wird zur Zeit mit sogenannten Pinhole-Kollimatoren mit kleinen Blendendurchmessern erzielt, allerdings ist die Empfindlichkeit einer solchen Kamera relativ gering. Zur Steigerung der Empfindlichkeit können prinzipiell mehrere Kameras simultan eingesetzt werden, allerdings stößt man hierbei - bedingt durch die relativ großen Dimensionen der Kamaras - schnell an geometrische Grenzen.

Aus der US 3,855,473 sind Szintillatorkristalle mit einer sich nach oben erweiterenden Abschirmung, optoelektronischen Wandlern mit nachgeschalteten Verstärkern und einer Auswerteeinheit bekannt, wobei die Szintillatorkristalle einseitig offene Hohlkörper sind. Eine hohe Ortsauflösung ist damit jedoch nicht möglich.

Des weiteren ist aus der US-A-4,348,591 eine Gamma-Kamera der gattungsgemäßen Art bekannt, die wegen des als Vertiefung ausgebildeten Pinhole-Kollimators ein sehr schmales Gesichtsfeld aufweist.

Aufgabe der Erfindung ist es, eine Gamma-Kamera der e. g. Art so auszugestalten, daß sie bei kleiner Abmessung ein erweitertes Gesichtsfeld.

Gelöst wird diese Aufgabe durch die kennzeichnenden Merkmale des Patentanspruchs 1. Die Unteransprüche beschreiben vorteilhafte Ausgestaltungen der Gamma-Kamera, bzw. nennen ein erweitertes Gesichtsfeld aufweist.

Die erfindungsgemäße Gamma-Kamera mit Bohrloch-Kristall und Pinhole-Kollimator bietet im Vergleich zur herkömmlichen Pinhole-Kamera ein erweitertes Gesichtsfeld, einen geringeren Meßabstand und - damit verbunden - eine höhere Empfindlichkeit. Bedingt durch die kompakte Bauweise können mehrere Bohrloch-Kameras leicht zu einem hochempfindlichen Array zusammengefaßt werden, mit dem auch dreidimensionale Darstellungen derAktivitätsverteilung im Meßobjekt erzeugt werden können. Der Vorteil gegenüber einer Anordnung mehrerer Kameras in Pinhole-Kollimator-Technik besteht darin, daß entsprechend der Anzahl der verwendeten Bohrloch-Kameras größere Impulsraten verarbeitet werden können und daß bei vergleichbarem Ortsauflösungsvermögen eine höhere Empfindlichkeit erzielt werden kann. Dies ist insbesondere bei dynamischen Untersuchungen sehr vorteilhaft. Der Vorteil gegenüber SPECT besteht darin, daß die Tomographie in fester Geometrie durchgeführt werden kann. Auch dies ist bei dynamischen Untersuchungen sehr vorteilhaft.

Die Erfindung wird im folgenden anhand eines Ausführungsbeispiels mit Hilfe der Figuren näher erläutert. Dabei zeigt die Fig. 1 schematisch einen Querschnitt durch die Kamera, der senkrecht zur Längsachse 6 des Kristallbohrlochs geführt ist, und die Fig. 2 schematisch einen Längsschnitt durch die Kamera. Die Fig. 3 zeigt ein aus mehreren Kameras bestehendes System.

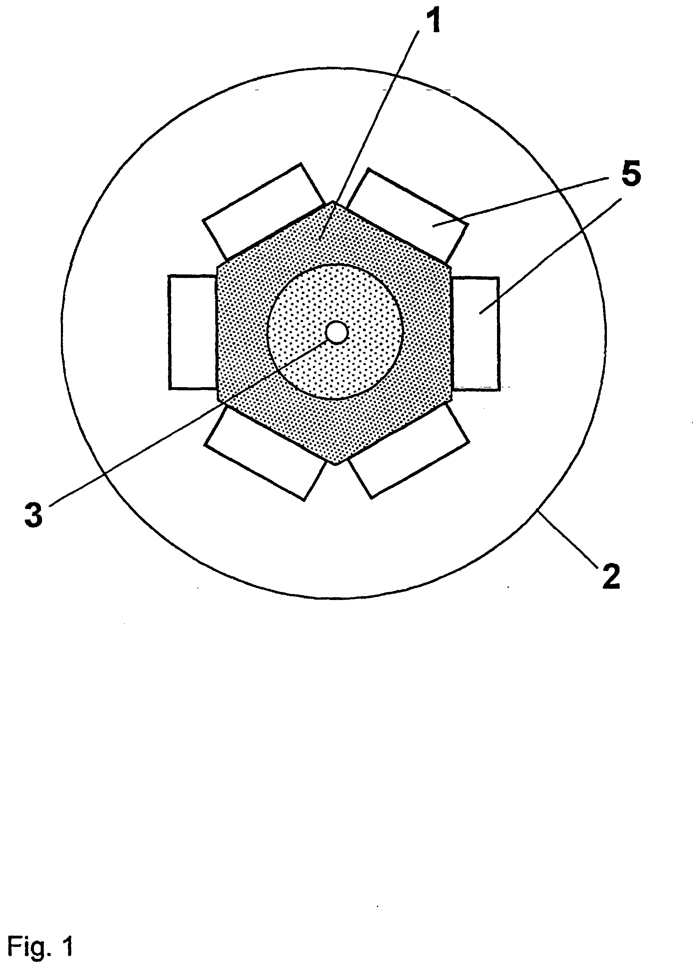

Die Fig. 1 zeigt als Kernstück der Kamera einen zylindrischen Bohrlochkristall 1, dessen Außenmantel sechskantig angeschliffen ist. Als Materialien für den Bohrlochkristall kommen alle Substanzen in frage, die durch radioaktive Strahlung zur Lichtemission angeregt werden, wie z. B. NaI(Tl) und CsI(Tl).

Das Bohrloch 1 hat - je nach dem angestrebten Gesichtsfeld und dem angestrebten Ortsauflösungsvermögen - einen Durchmesser von etwa 2 cm bis zu etwa 15 cm und eine Tiefe von etwa 2 cm bis zu etwa 30 cm. Die Wandstärke des Bohrlochkristalls ist so gering wie herstellungstechnisch möglich (mindestens etwa 1 cm).

An den sechs angeschliffenen Außenflächen des Kristalls 1 befinden sich sechs optoelektronische Wandler 5. Kollimator 2 und Blende 3 sind als Kreise dargestellt. Die Anzahl der Außenflächen kann zwischen 4 und 10 variiert werden.

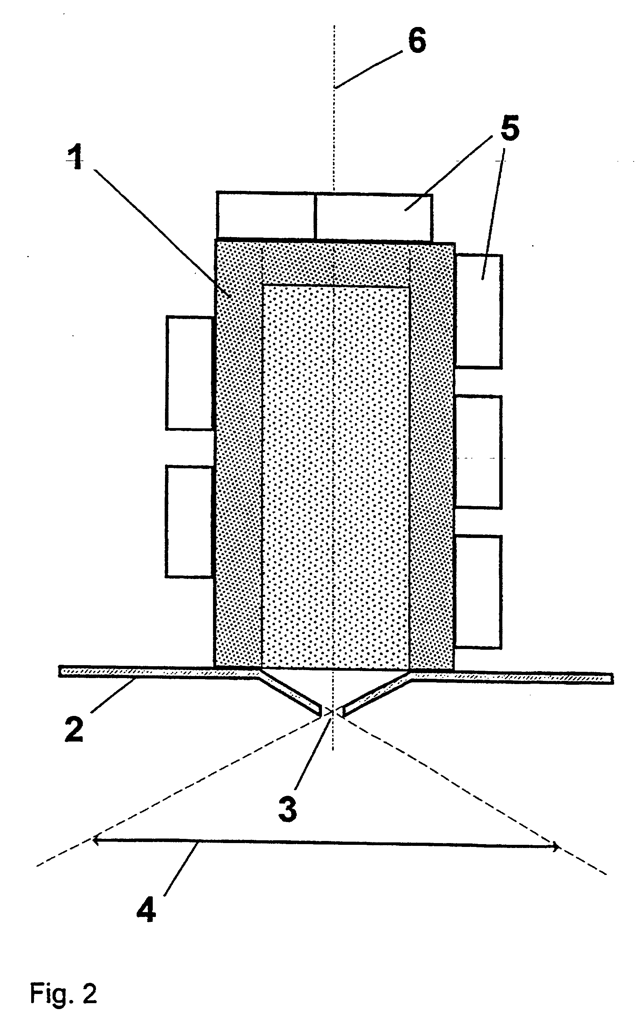

Die Fig. 2 zeigt außen an dem U-förmigen Querschnitt des Kristalls 1 im Wechsel zwei bzw. drei übereinander angeordnete optoelektronische Wandler 5 geeigneter Größe. Die Achse 6 ist die Symmetrieachse des Kristalls 1. Auf der Oberseite des Kristalls befinden sich drei optoelektronische Wandler 5 (hier sind nur zwei dargestellt), die achsensymmetrisch jeweils über denjenigen Kristallsegmenten angeordnet sind, an deren Außenwand nur zwei optoelektronische Wandler 5 angebracht sind. Damit wird für nahezu alle Raumpunkte im Kristallvolumen eine 2-Abdeckung mit Photomultipliern erzielt. Bei längeren Kristallen sind selbstverständlich noch mehr optoelektronische Wandler 5 vorzusehen. Der Pfeil 4 markiert den Öffnungswinkel des Gesichtsfeldes.

Nach unten wird der Kristall von einem Pinhole-Kollimator 2, abgeschlossen, der im Bereich des Bohrlochs kegelförmig ausgewölbt ist. Im Zentrum des Kollimators befindet sich eine Blende 3, deren Durchmesser - je nach angestrebtem Ortsauflösungsvermögen - zwischen etwa 1 mm und 5 mm beträgt. Das Gesichtsfeld hat - je nach Ausformung des Kollimators 2 einen Öffnungswinkel bis zu etwa 120°.

Die Strahleneinfallsrichtung wird wie bei der herkömmlichen Pinhole-Kamera durch die vom Ort der Szintillation und vom Mittelpunkt der Kollimatorblende definierte Gerade bestimmt.

Die Bestimmung des Szintillationsortes erfolgt dabei durch einen Vergleich der von den verschiedenen Photomultipliern registrierten Lichtintensitäten. Dabei kann - im Gegensatz zur herkömmlichen Pinhole-Kamera - prinzipiell eine dreidimensionale Bestimmung des Szintillationsortes durchgeführt werden. Dies führt insbesondere bei großen Einfallswinkeln zu einer wesentlich besseren Bestimmung der Strahleneinfallsrichtung.

Mit der hier beschriebenen Gamma-Kamera können wie mit der herkömmlichen Pinhole-Kamera zweidimensionale Bilder erzeugt werden, indem die gemessene Richtungsverteilung der einfallenden Strahlung in eine vorgegebene Objektebene projiziert wird. Die Bildqualität ist dabei umso besser, je geringer der mittlere Abstand der Nukliddeposition von dieser Projektionsebene ist.

Mit einem Array von mehreren Bohrloch-Kameras können darüber hinaus auch dreidimensionale Darstellungen der Aktivitätsverteilung erzeugt werden, indem man die von den verschiedenen Kameras gemessenen Richtungsverteilungen ähnlich wie bei der 7-Segment-Pinhole-Kollimator-Technik oder der single-Photon-Emission-Computer-Tomography (SPECT) entfaltet (Hermann, H.J., Nuklearmedizin, Verlag Urban & Schwarzenberg München, 3. Auflage 1992, S. 64-66 bzw. S. 55-58)

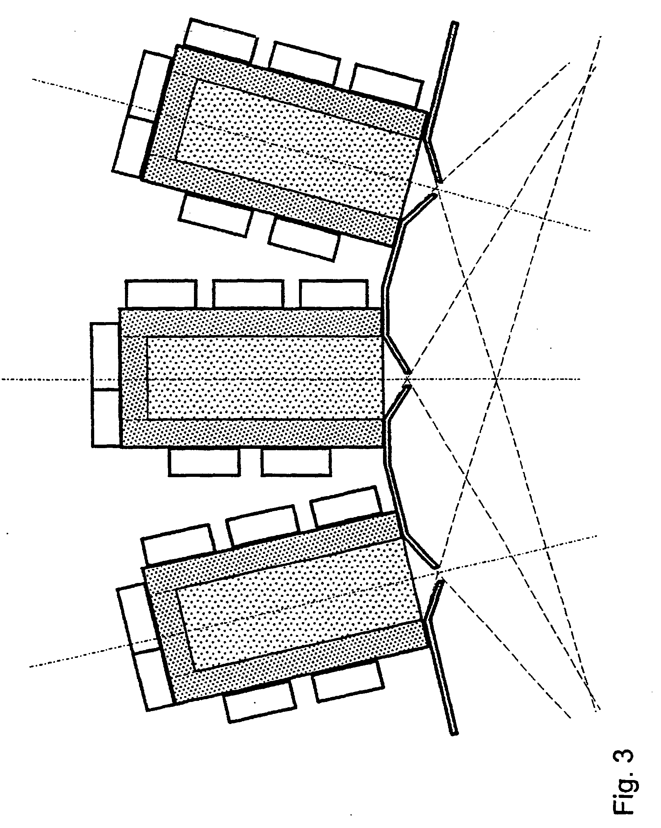

Die Fig. 3 zeigt als Anwendungsbeispiel ein aus sieben Bohrloch-Kameras aufgebautes Photonen-Emissions-Computer-Tomographie-System. Um eine zentrale Kamera sind radial sechs weitere Kameras angeordnet. Die Symmetrieachsen 6 der sechs peripheren Kameras stehen in einem Winkel zur Symmetrieachse der zentralen Kamera, so daß sich eine großräumige Überschneidung der Gesichtsfelder der einzelnen Kameras ergibt. Die Bohrlöcher der Kristalle 1 haben in diesem Beispiel einen Durchmesser von etwa 6 cm und eine Tiefe von etwa 15 cm. Das Tomographie-System hat damit im Meßabstand von 15 cm ein Gesichtsfeld mit einem Durchmesser von etwa 60 cm.

Als optoelektronische Wandler können Photomultiplier oder auch dünne Photodioden zur Registrierung des Szintillationslichts verwendet werden. Bei letzteren empfiehlt sich allerdings die Verwendung von CsI(Tl)-Kristallen, da die spektrale Verteilung des Szintillationslichts bei diesem Material besser mit dem spektralen Ansprechvermögens der Photodioden übereinstimmt.

Bei Verwendung von Photodioden ist eine noch kompaktere Bauweise möglich, so daß prinzipiell noch mehr Kamera-Module zu einem Tomographie-System zusammengefaßt werden können. Allerdings ist der Rausch-Signal-Abstand bei den heute erhältlichen Photodioden geringer als bei den Photomultipliern, so daß der Gewinn hinsichtlich Ortsauflösungsvermögen und Empfindlichkeit noch nicht abgeschätzt werden kann.

### Bezugszeichenliste:

- 1.: Kristall
- 2.: Kollimator
- 3.: Lochblende
- 4.: Gesichtsfeld
- 5.: Optoelektronische Wandler
- 6.: Detektorachse

## Patentansprüche

1. Gamma-Kamera bestehend aus einem Szintillatorkristall mit Pinhole-Kollimator, mindestens vier optoelektronischen Wandlern mit nachgeschalteten Verstärkern und einer Auswerteeinheit, **dadurch gekennzeichnet, daß** der Szintillatorkristall (1) ein einseitig offener Hohlkörper ist, dessen Öffnung durch den Pinhole-Kollimator (2) abgedeckt ist, wobei der Pinhole-Kollimator (2) im Bereich des Bohrlochs kegelförmig ausgewölbt ist und eine Blende (3) im Zentrum aufweist.

2. Gamma-Kamera nach Anspruch 1, **dadurch gekennzeichnet, daß** der Hohlkörper ein Zylinder mit einem konzentrischen zylindrischen Bohrloch ist.

3. Gamma-Kamera nach Anspruch 1, **dadurch gekennzeichnet, daß** der Hohlkörper ein Zylinder mit einem konzentrischen zylindrischen Bohrloch ist, dessen Außenmantel aus ebenen Flächen besteht.

4. Gamma-Kamera nach Anspruch 1, **dadurch gekennzeichnet, daß** der Hohlkörper aus planparallelen Segmenten besteht.

5. Verwendung von mehreren Gamma-Kameras gemäß einem der Ansprüche 1 bis 4 zur Tomographie.

## Claims

1. Gamma camera, comprising a scintillator crystal with a pin-hole collimator, at least four optoelectronic transducers with amplifiers connected to the output side thereof and an evaluation unit, **characterised in that** the scintillator crystal (1) is a hollow body which is open at one end, the opening of which hollow body is covered by the pin-hole collimator (2), the pin-hole collimator (2) tapering outwardly in the region of the pin hole and having a diaphragm (3) in the centre.

2. Gamma camera according to claim 1, **characterised in that** the hollow body is a cylinder with a concentric cylindrical pin hole.

3. Gamma camera according to claim 1, **characterised in that** the hollow body is a cylinder with a concentric cylindrical pin hole, the external casing of which comprises flat faces.

4. Gamma camera according to claim 1, **characterised in that** the hollow body comprises plane-parallel segments.

5. Use of a plurality of gamma cameras according to one of claims 1 to 4 for tomography.

## Revendications

1. Caméra gamma composée d'un cristal de scintillation avec un collimateur en trou d'épingle, d'au moins quatre convertisseurs optoélectroniques suivis d'amplificateurs et d'une unité de traitement,
**caractérisée en ce que**
le cristal du scintillateur (1) est un corps creux ouvert d'un côté dont l'ouverture est couverte par un collimateur en trou d'épingle (2),
le collimateur en trou d'épingle (2) ayant une forme bombée en tronc de cône au niveau du trou de perçage et comporte en son centre un diaphragme (3).

2. Caméra gamma selon la revendication 1,
**caractérisée en ce que**
le corps creux est un cylindre avec un perçage cylindrique concentrique.

3. Caméra gamma selon la revendication 1,
**caractérisée en ce que**
le corps creux est un cylindre avec un perçage cylindrique concentrique dont l'enveloppe extérieure est formée d'une surface très plane.

4. Caméra gamma selon la revendication 1,
**caractérisée en ce que**
le corps creux est formé de segments plans parallèles.

5. Application de plusieurs caméras gamma selon l'une quelconque des revendications 1 à 4 à la tomographie.
